# EUROPEAN PATENT SPECIFICATION

(11) **EP 2 496 185 B1**
(45) Date of publication and mention of the grant of the patent: **10.06.2015**
(21) Application number: 09752956.4
(22) Date of filing: 05.11.2009
(51) Int. Cl.: A61F 2/28, A61F 2/30, A61F 2/44, A61F 2/46

(54) **SELF-PIVOTING SPINAL IMPLANT AND ASSOCIATED INSTRUMENTATION**
SELBSTDREHENDES WIRBELSÄULENIMPLANTAT UND ZUGEHÖRIGE INSTRUMENTE
IMPLANT RACHIDIEN AUTO-ROTATIF ET INSTRUMENTS ASSOCIÉS

(43) Date of publication of application: 12.09.2012
(73) Proprietor: Synthes GmbH, 4436 Oberdorf (CH)
(72) Inventor: LINDENMANN, Philippe, CH-4053 Basel (CH); SAIDHA, Sean, CH-4059 Basel (CH); BAUDOUIN, Cyril, F-68400 Riedisheim (FR); FATONE, Peter, Exton MA 19341 (US)
(74) Representative: Klunker . Schmitt-Nilson . Hirsch
(86) International application number: PCT/US2009/063371
(87) International publication number: WO 2011/056172

(56) References cited:
- WO-A1-2007/070751
- WO-A2-2008/036636
- US-A1- 2006 106 460
- US-A1- 2006 229 627
- US-A1- 2007 282 441
- US-A1- 2008 119 935
- US-A1- 2008 306 488

## Description

### BACKGROUND OF THE INVENTION

The unilateral transforaminal insertion of an interbody spacer for lumbar spinal fusion presents challenges to the surgeon tasked with the procedure due to the curved manipulation path that the implant must undergo once it enters the disc space. The procedure presents a further challenge of coupling the implant to the inserter instrument while allowing the implant a limited amount of rotation or articulation to follow the desired path. These challenges also present themselves to other angular unilateral approaches to the spine, in which the initial access corridor is linear yet, once the implant enters the disc space, the implant must be manipulated or articulated along a curved path. Conventional transforaminal lateral interbody fusion (TLIF) implants, for example, are inserted using a combination of a linear insertion path and a hammering of the implant into the desired position using pushers that provide the desired anterior positioning of the implant. Alternately, a stepwise straight hammering process alternating with an active turning technique is often used to manipulate the implant from the entry position to the final desired position. The conventional TLIF and other angular unilateral systems and insertion methods fail to provide implants, instrumentation, and methods that allow the implant to be easily inserted to its final desired position within the disc space.

It is therefore desired to provide a spinal implant and associated instrument and method that improves the ease with which the implant may be manipulated during insertion or once within the disc space.

US 2006/0106460 A1 discloses an intervertebral implant having a body with curved, substantially parallel posterior and anterior surfaces. The implant has longitudinal and lateral channels that can be packed with bone graft. A slot for engagement with an instrument is provided at an engagement end. Similarly, US 2008/0306488 A1 discloses an intervertebral implant that has apertures at an engagement end for engagement with an instrument that has a pivoting mechanism to allow pivoting of the implant. A further intervertebral implant is .disclosed in US 2007/0282441 A1 that has lateral slots for secure engagement with an insertion instrument. In WO 2008/036636 A2 an intervertebral implant is disclosed that has a cylindrical post in a slot at an engagement end to allow pivoting of the implant. Teeth are provided at an edge of the implant to fix the orientation of the implant relative to the insertion instrument.

### BRIEF SUMMARY OF THE INVENTION

Briefly stated, a first embodiment of the present invention comprises an intervertebral implant including an insertion end, an opposing engagement end, and first and second opposed main surfaces configured to contact respective adjacent vertebral endplates. Each of the first and second main surfaces has an anterior edge, a posterior edge, and extends between the insertion and engagement ends. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from at least one of the anterior and posterior walls and extending at least partially between the first and second main surfaces. The post includes a plurality of exposed facets and is configured for pivotable engagement with an insertion instrument.

Another embodiment of the present invention comprises an intervertebral implant including an insertion end, an opposing engagement end, and first and second opposed main surfaces configured to contact respective adjacent vertebral endplates. Each of the first and second main surfaces has an anterior edge, a posterior edge, and extends between the insertion and engagement ends. Each anterior edge has a generally linear portion proximate the engagement end and a generally concave portion, and each posterior edge has a generally linear portion proximate the engagement end and a generally convex portion. The generally linear portion of the anterior edge converges with the generally linear portion of the posterior edge at the engagement end for each of the first and second main surfaces. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from at least one of the anterior and posterior walls and extending at least partially between the first and second main surfaces. The post includes a plurality of exposed facets and is configured for engagement with a pivotable insertion instrument.

Still another embodiment of the present invention comprises an intervertebral implant including an insertion end, an opposing engagement end, and first and second opposed main surfaces configured to contact respective adjacent vertebral endplates. Each of the first and second main surfaces has an anterior edge, a posterior edge, and extends between the insertion and engagement ends. Each anterior edge is generally concave and each posterior edge is generally convex. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from the anterior and posterior walls and extending at least partially between the first and second main surfaces. The post includes a plurality of facets disposed around an entire periphery thereof and is configured for engagement with a pivotable insertion instrument. At least one abutment surface is disposed within the slot distally from the post. The at least one abutment surface limits rotation of the implant about the post when the post is engaged with the pivotable insertion instrument.

Yet another embodiment of the present invention comprises an intervertebral implant including an insertion end, an opposing engagement end, and first and second opposed main surfaces configured to contact respective adjacent vertebral endplates. Each of the first and second main surfaces has an anterior edge, a posterior edge, and extends between the insertion and engagement ends. Each anterior edge is generally concave and each posterior edge is generally convex. An axial bore is formed between the anterior and posterior edges and extends between the first and second main surfaces. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends at least partially along the anterior and posterior walls. A post is positioned within the slot and extends at least partially between the first and second main surfaces. The post includes a plurality of facets and is configured for engagement with a pivotable insertion instrument. The intervertebral implant also includes a plurality of markers. At least one of the markers extends between the first and second main surfaces within one of the anterior and posterior walls. At least one other of the markers is disposed generally transverse to the at least one of the markers and extends from the insertion end toward the axial bore.

It is disclosed a method for implanting an intervertebral implant into a disc space disposed between first and second endplates of adjacent vertebral bodies of a patient. The method includes providing an access corridor to a spinal level in need, removing at least a portion of disc material between the adjacent vertebra, and providing an interbody spacer implant. The implant includes an insertion end, an opposing engagement end, and first and second opposed main surfaces configured to contact the respective first and second vertebral endplates. Each of the first and second main surfaces has an anterior edge, a posterior edge, and extends between the insertion and engagement ends. Each anterior edge is generally concave and each posterior edge is generally convex. Each of the first and second main surfaces includes a plurality of curved parallel ridges protruding from the respective surface and extending from the insertion end to the engagement end. Each of the plurality of parallel ridges includes a plurality of teeth. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from at least one of the anterior and posterior walls and extending at least partially between the first and second main surfaces. The post includes a plurality of exposed facets and is configured for engagement with a pivotable insertion instrument. The method also includes providing an insertion instrument. The instrument includes a proximal end, a distal end, and a longitudinal axis therebetween, and an inner member and an outer member. The inner member is movable along the longitudinal axis with respect to the outer member. The inner member has a grasping portion at the distal end. The grasping portion includes a plurality of facet surfaces configured for engagement with the plurality of the post facets. The method also includes inserting the grasping portion of the instrument into the slot of the implant such that the grasping portion surrounds the post, engaging the post of the implant with the grasping portion of the instrument such that the post is rotationally fixed with respect to the grasping portion, inserting the implant using the instrument through the access corridor until at least the insertion end is introduced into the at least partially cleared out disc space and such that the at least a portion of the ridges of the first and second main surfaces contact the first and second verebtral endplates, respectively, adjusting the instrument such that the post of the implant remains engaged with the grasping portion of the instrument but rotation of the post is permitted within the grasping portion, delivering impaction forces to the proximal end of the instrument such that the post of the implant articulates with respect to the grasping portion of the instrument and the implant is guided by vertebral rails into a desired position, releasing the post of the implant from the grasping portion of the instrument, and withdrawing the instrument through the access corridor.

Yet another embodiment of the present invention comprises a system for spine surgery at a disc space disposed between first and second endplates of adjacent vertebral bodies of a patient. The system includes an intervertebral implant including an insertion end and an opposing engagement end. First and second opposed main surfaces are configured to contact respective adjacent vertebral endplates. Each of the first and second main surfaces has an anterior edge, a posterior edge, and extends between the insertion and engagement ends. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from at least one of the anterior and posterior walls and extending at least partially between the first and second main surfaces. The post includes a plurality of exposed facets. A trial implant includes an insertion end and an opposing engagement end. First and second opposed main surfaces are configured to contact respective adjacent vertebral endplates. Each of the first and second main surfaces has an anterior edge, a posterior edge, and extends between the insertion and engagement ends. An anterior wall is formed between the first and second main surfaces and along the anterior edges thereof. A posterior wall is formed between the first and second main surfaces and along the posterior edges thereof. The anterior wall and the posterior wall converge at the insertion and engagement ends. A slot is formed at the engagement end and extends continuously between and at least partially along the anterior and posterior walls. A post is positioned within the slot, spaced from at least one of the anterior and posterior walls and extending at least partially between the first and second main surfaces. The post includes a plurality of exposed facets. An insertion instrument includes a proximal end, a distal end, a longitudinal axis therebetween, an inner member, and an outer member. The inner member is translatable with respect to the outer member along the longitudinal axis and has a grasping portion at the distal end. The grasping portion includes a plurality of facet surfaces engagable with the plurality of the post facets of the intervertebral implant and the trial implant. The instrument has a first configuration in which the grasping portion assumes an open configuration for allowing coupling of the instrument to the post of one of the intervertebral implant and the trial implant, a second configuration in which the instrument is securely coupled to the post of one of the intervertebral implant and the trial implant while allowing the post to rotate within the grasping portion under a given force, and a third configuration wherein the instrument is securely coupled to the post of one of the intervertebral implant and the trial implant while preventing rotation of the post with respect to the grasping portion.

### BRIEF DESCRIPTION OF THE SEVERAL VIEWS OF THE DRAWINGS

The foregoing summary, as well as the following detailed description of preferred embodiments of the instrument of the present application, will be better understood when read in conjunction with the appended drawings. For the purposes of illustrating the self-pivoting spinal implant and the associated instrumentation of the present application, there is shown in the drawings preferred embodiments. It should be understood, however, that the application is not limited to the precise arrangements and instrumentalities shown. In the drawings:
Fig. 1 is a rear perspective view of a self-pivoting TLIF implant in accordance with a first preferred embodiment of the present invention;
Fig. 2 is a front perspective view of the self-pivoting TLIF implant of Fig. 1;
Fig. 3 is a top plan view of the self-pivoting TLIF implant of Fig. 1;
Fig. 4 is a front and left side perspective view of the self-pivoting TLIF implant of Fig. 1;
Fig. 5 is a front perspective view of the self-pivoting TLIF implant of Fig. 1 and a bone growth promoting material configured for insertion into the implant;
Fig. 6 is a front perspective view of the self-pivoting TLIF implant of Fig. 1 showing a preferred arrangement of radiopaque markers;
Fig. 7 is a partial front perspective cross-sectional view of an inserter instrument in accordance with a first preferred embodiment of the present invention, the inserter instrument shown in open configuration;
Fig. 8A is a top plan view of the inserter instrument of Fig. 7;
Fig 8B is a partial front perspective view of the inserter instrument of Fig. 7 in the open configuration;
Fig. 9A is a top plan view of the inserter instrument of Fig. 7 in an initial articulation position and in a finally locked configuration;
Fig. 9B is a cross-sectional view of the inserter instrument of Fig. 7 in the initial articulation position and in the finally locked configuration;
Fig. 9C is a cross-sectional view of the inserter instrument of Fig. 7 in the initial articulation position and in a provisionally locked configuration;
Fig. 9D is a cross-sectional view of the inserter instrument of Fig. 7 in a final articulation position and in the provisionally locked configuration;
Fig. 10A is a cross-sectional view of the inserter instrument of Fig. 7 in the final articulation position and in the finally locked configuration;
Fig. 10B is a top plan view of the inserter instrument of Fig. 7 in the final articulation position and in the finally locked configuration;
Fig. 10C is a cross-sectional view of the inserter instrument of Fig. 7 in the final articulation position and in the open configuration;
Fig. 11A is a top plan view, partially broken away, of one position of the implant of Fig. 1 and the instrument of Fig. 7 with respect to a disc space, partially broken away, as the implant is inserted therein;
Fig. 11B is a top plan view, partially broken away, of a the implant and instrument of Fig. 11A in a second position;
Fig. 11C is a top plan view, partially broken away, of a the implant and instrument of Fig. 11B in a third position;
Fig. 11D is a top plan view of a the implant and instrument of Fig. 11C in a fourth position;
Fig. 12A is a rear perspective view of a trial implant in accordance with one embodiment of the present invention;
Fig. 12B front perspective view of the trial implant of Fig. 12A;
Fig. 12C is a rear elevational view of the trial implant of Fig. 12A; and
Fig. 12D is a left side elevational view of the trial implant of Fig. 12A.

### DETAILED DESCRIPTION OF THE INVENTION

Certain terminology is used in the following description for convenience only and is not limiting. The words "right," "left," "lower," and "upper" designate directions in the drawings to which reference is made. The words "inwardly" or "distally" and "outwardly" or "proximally" refer to directions toward and away from, respectively, the patient's body, or the geometric center of the interbody spacer implant and related parts thereof. The words, "anterior," "posterior," "superior," "inferior," and related words and/or phrases designate preferred positions and orientations in the human body to which reference is made and are not meant to be limiting. The terminology includes the above-listed words, derivatives thereof and words of similar import.

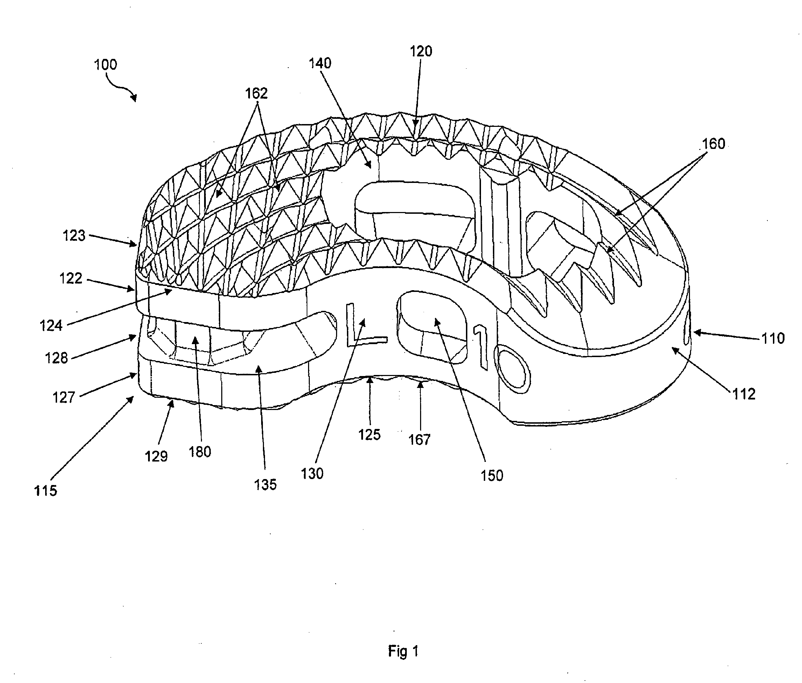

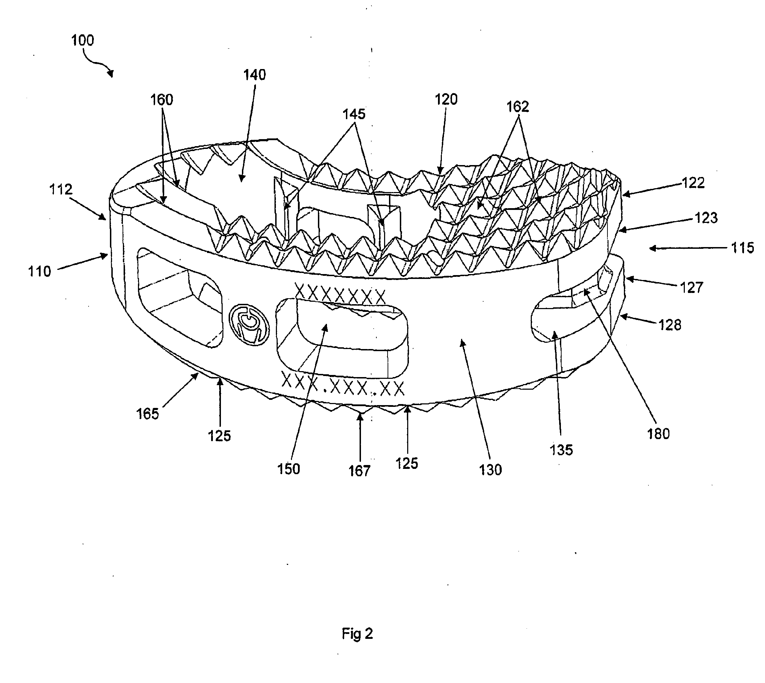

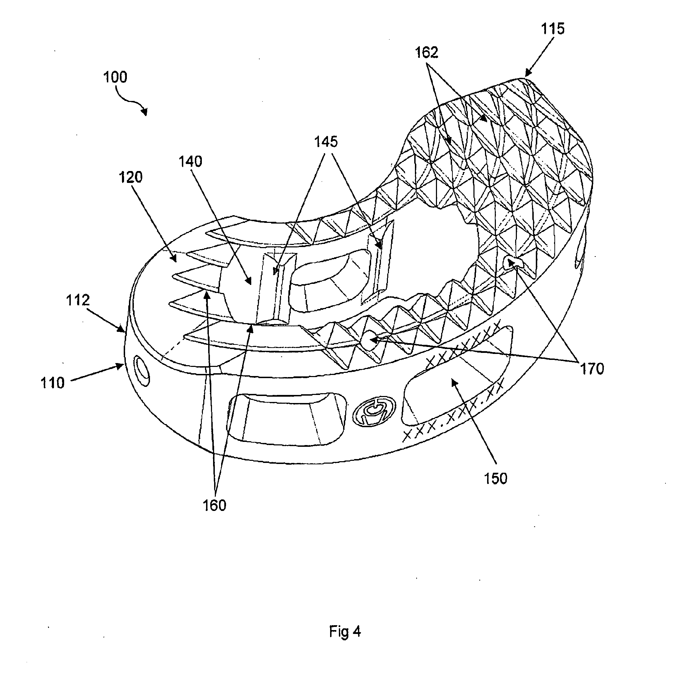

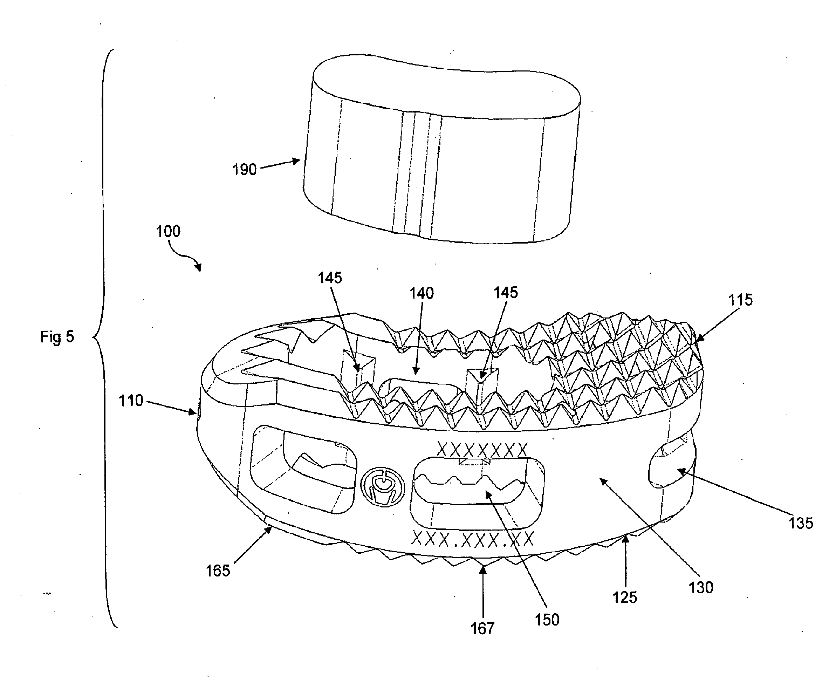

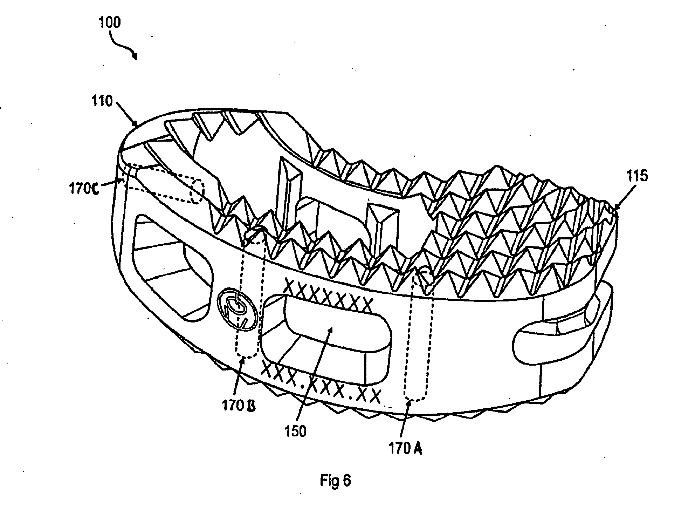

Referring to Figs. 1-6, a TLIF spacer 100 is provided that includes an insertion end 110 and an engagement end 115, the insertion end 110 preferably forming a bullet-nose 112 or having some other tapered geometry for enhancing the ease of insertion and/or for applying a distraction force to the two vertebral bodies between which the implant 100 is configured to be inserted. The implant 100 further includes a first main or superior surface 120 that is configured for contacting the inferior endplate of a superior vertebral body and a second main or inferior surface 125 that is configured for contacting the superior endplate of an inferior vertebral body. One or more walls 130 on anterior and posterior sides extend between the superior and inferior surfaces 120, 125 and enclose an axial bore 140 that extends through both the superior and inferior surfaces 120, 125. The axial bore 140 is configured to house a bone graft 190 or other fusion enhancing material.

One or more lateral windows 150 are disposed in the walls 130 and provide a visibility window for observing the fusion occurring between the vertebral bodies and enhancing the vascularization of the bone graft 190 disposed within the axial bore 140 to assist fusion, as well as to increase the volume of the axial bore 140. One or more surface features 145 are provided along interior portions of the walls 130 that form the axial bore 140 to assist in securing the bone graft 190 within the axial bore 140. The features 145 can assume the form of one or more ridges extending through the axial bore 140 along the cranial-caudal direction, grooves, or other surface texturing that enhances the friction between the bone graft 190 and the interior of the walls 130 that form the axial bore 140.

In a first preferred embodiment, the TLIF spacer 100 has a kidney bean or banana shape having a curvilinear geometry between its insertion and engagement ends 110, 115. This shape may be accomplished by having an anterior edge of the superior and inferior surfaces 120, 125 along with the anterior wall 130 be generally concave and a posterior edge of the superior and inferior surfaces 120, 125 along with the posterior wall 130 be generally convex. However, a variety of geometries may be utilized for the implant 100, depending on the desired amount of surface contact between the endplates of the vertebral bodies and the implant 100, the number of implants 100 desired to be implanted within the disc space (e.g., one or two), the approach chosen for the surgery, the desired location of the implant within the disc space (anterior or posterior), or the like. Disposed upon the superior surface 120 adjacent the insertion end 110 are a plurality of curvilinear superior ridges 160 that are arranged parallel to one another along the curvature of the TLIF implant 100.

In a first preferred embodiment, the superior ridges 160 include two linearly sloped surfaces that meet to form an apex. As the superior ridges 160 extend along their curvilinear path away from the insertion end 110, the superior ridges 160 are interrupted to form a plurality of superior teeth 162. The superior teeth 162 are disposed at the engagement end 115 and along at least a portion of anterior and posterior sides of the axial bore 140. Similarly, disposed upon the inferior surface 125 adjacent the insertion end 110 is a plurality of curvilinear inferior ridges 165 that are arranged parallel to one another along the curvature of the TLIF implant 100. As the inferior ridges 165 extend along their curvilinear path away from the insertion end 110, the inferior ridges 165 are interrupted to form a plurality of inferior teeth 167. The inferior teeth 167 are disposed at the engagement end 115 and on the anterior and posterior sides of the axial bore 140. The superior and inferior ridges 160, 165 guide the insertion of the TLIF implant 100 under the compressive forces of the adjacent vertebral bodies, while the superior and inferior teeth 162, 167 assist in the primary fixation of the TLIF implant 100.

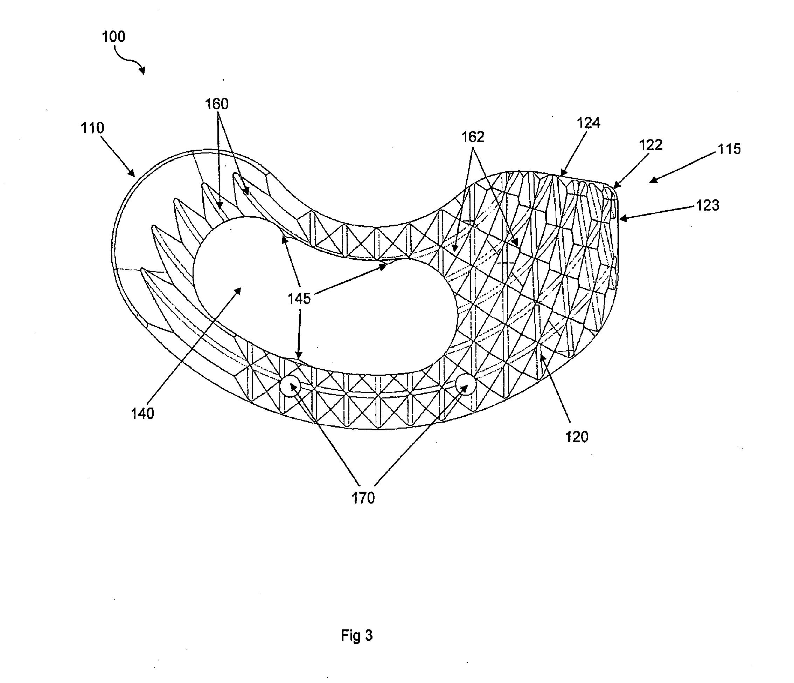

Referring to Figs. 3, 4, and 6, one or more radiopaque markers 170, made from material capable of radiographical imaging, such as pins or beads of stainless steel, titanium, tantalum, titanium-aluminum-niobium (TAN), or the like, are included in the TLIF implant 100 for enabling visualization and controlling of the position of the TLIF implant 100 during and after insertion into the disc space. In a first preferred embodiment, the markers 170 are elongated and include a first marker 170A, a second marker 170B, and a third marker 170C. The first and second markers 170A, 170B are disposed in the cranial-caudal direction on either side of the lateral window 150 within the anterior wall 130 of the implant 100. The third marker 170C is disposed proximate the insertion end 110, with a longitudinal axis thereof extending from the insertion end 110 toward the axial bore 140.

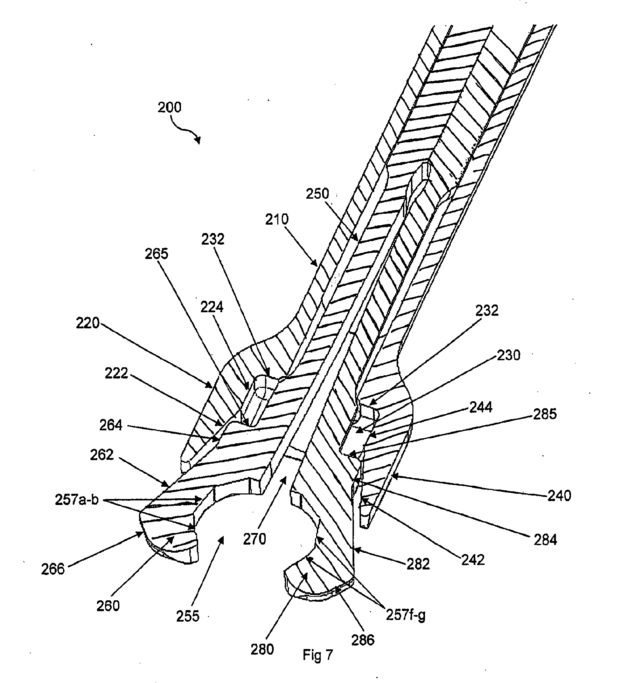

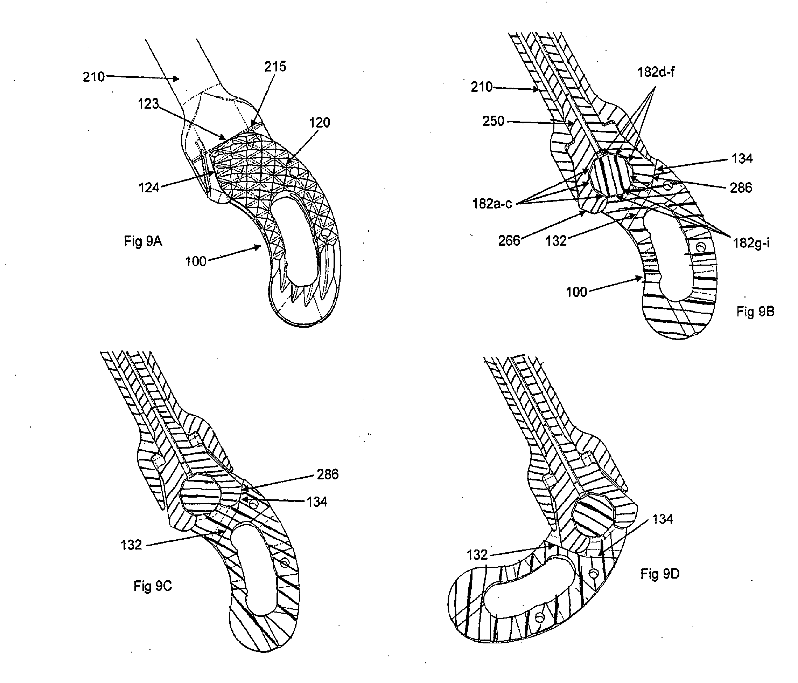

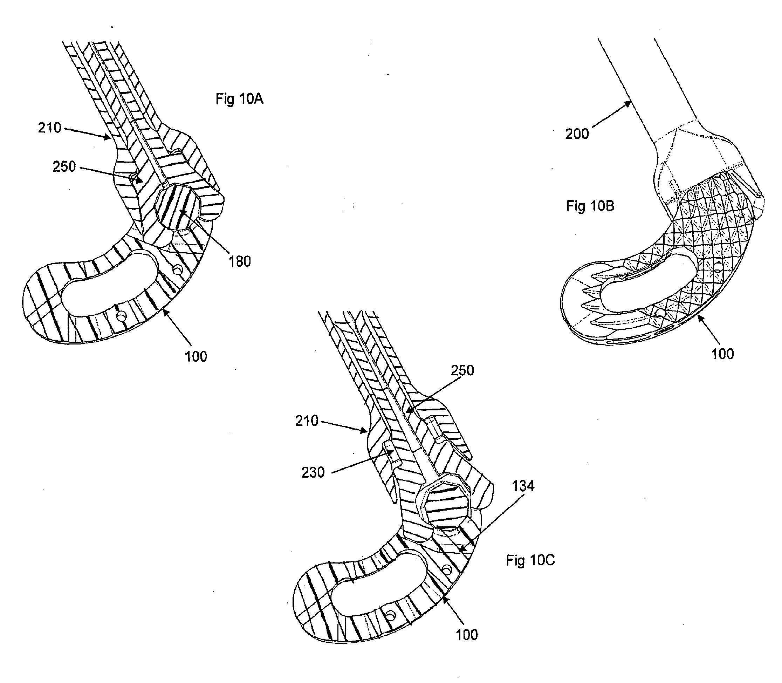

The engagement end 115 is characterized by the absence of the walls 130 extending fully between the superior and inferior surfaces 120, 125. That is, a slot 135 is formed at the engagement end 115 that extends continuously between and at least partially along the anterior and posterior walls 130. A post 180 is positioned within the slot 135, which is spaced apart from the anterior and posterior walls 130 and extends at least partially between the superior and inferior surfaces 120, 125 and serves as an instrument engagement feature. Adequate space is provided by the slot 135 for the engagement portion of an instrument 200 (Fig. 7) to engage the post 180. As shown in Figs. 9 and 10, within the implant 100, the walls 130 disposed between the axial bore 140 and the post 180 include first and second mating surfaces 132, 134 facing the post 180 between which an obtuse angle is formed for providing a pair of mechanical stops to the range of allowable articulation of the implant 100 with respect to the instrument 200. The first and second mating surfaces 132, 134 are preferably linear surfaces, but may also be curved or the like. Alternatively, stop pins or the like may be used to limit articulation of the implant 100.

Referring now to Figs. 9 and 10, in a first preferred embodiment, the post 180 is polygonal in cross-section and includes nine exposed facets 182a-182i arranged around an entire periphery thereof and extending in the cranial-caudal direction between the superior and inferior surfaces 120, 125. The facets 182a-182i are configured to enhance the engagement and interaction between the instrument 200 and the implant 100 during the insertion of the implant 100. Preferably, seven of the facets 182a-182f, 182i are flat surfaces, while the remaining two facets 182g-182h are curved surfaces. In an alternate embodiment, the post 180 may include a different polygonal number of facets 182. In yet another alternate embodiment, the post 180 can be cylindrical and thus include zero facets 182, and may include other features for governing the articulation of the implant 100 with respect to the instrument 200 during its insertion. For example, the post 180 can include dimples, teeth, surface texturing, grooves, or the like.

Referring now to Figs. 1-5 and 7, the engagement end 115 of the superior surface 120 terminates in a superior corner 122, which includes superior first and second flat segments 123, 124 originating near the post 180 and converging at an angle disposed proximate the engagement end 115 of the implant 100. Similarly, the engagement end 115 of the inferior surface 125 terminates in an inferior corner 127, which include inferior first and second flat segments 128, 129 originating near the post 180 and converging terminating at an angle disposed proximate the engagement end 115 of the implant 100. The superior first flat segment 123 and the inferior first flat segment 128 are configured to be engagable by a portion of the instrument 200, as is described in detail below, to provide a toggle-free connection, as are the superior second flat segment 124 and the inferior second flat segment 129. The rims of both the superior and inferior corner segments 122, 127 have a width extending a short distance from the superior and inferior surfaces 120, 125 toward the center of the implant 100. The surfaces of the rims are also flat for enhancing the interaction between the instrument 200 and the implant 100. The implant 100 can be formed from a variety of biocompatible materials, including but not limited to titanium, stainless steel, allograft bone, or polymers such as polyaryletheretherketone (PEEK) and polyetherketoneketone (PEKK), titanfoam, porous PEEK, or the like.

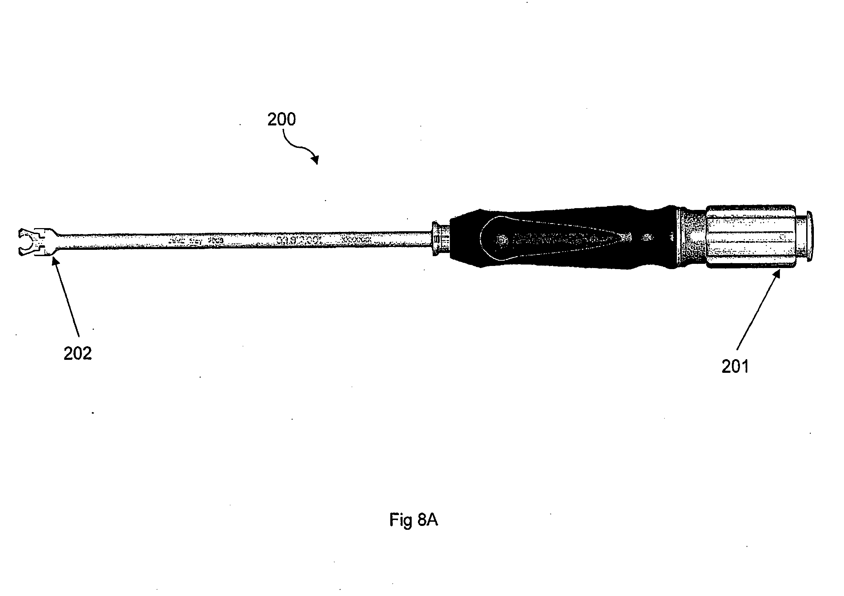

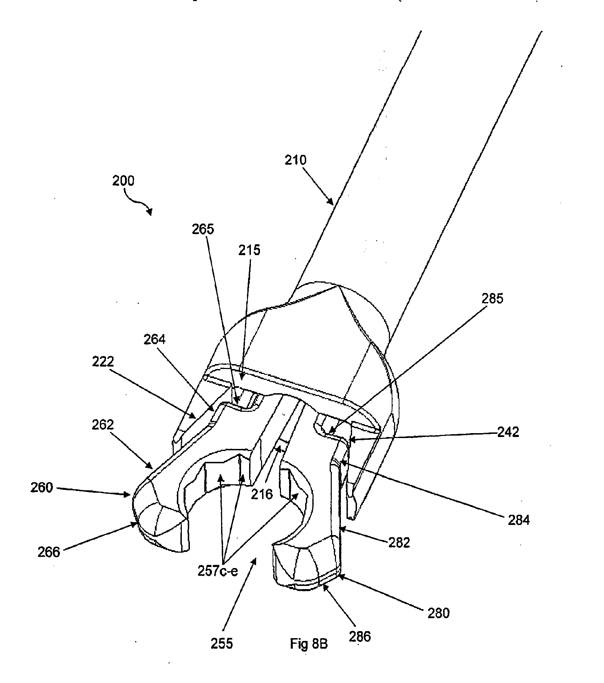

Referring to Figs. 7-8, an instrument 200 is provided that includes a longitudinal axis extending between a proximal end 201 and a distal end 202. The instrument 200 includes an elongated cannulated outer member 210 that surrounds an elongated inner member 250. The inner member 250 is configured to be translatable with respect to the outer member 210 along the longitudinal axis. Alternatively, the instrument 200 can be configured such that the outer member 210 is translatable with respect to the inner member 250 along the longitudinal axis to perform in the same manner. The proximal end of the outer member 210 includes a handle portion (not shown) and an actuation mechanism (not shown) for translating the inner member 250 with respect to the outer member 210. The distal end of the outer member 210 includes an outer member first arm 220 and an outer member second arm 240 that are separated by a gap 230 that forms the distal portion of the cannula. The gap 230 includes a pair of laterally-oriented surfaces 232 on either side of the cannula disposed at the proximal end of the outer member first and second arms 220, 240. The laterally-oriented surfaces 232 serve as a stop to the retraction of the inner member 250 with respect to the outer member 210. The interior surface of the first arm 220 includes an outer member first arm interior linear taper 222 disposed distal to an outer member first arm interior straight portion 224, while the interior surface of the second arm 240 includes an outer member second arm interior linear taper 242 disposed distal to an outer member second arm interior straight portion 244. The first and second arm interior linear tapers 222, 242 combine to form two wedging surfaces.

A laterally-extending superior exterior flat surface 215 of the outer member 210 is disposed between the distal ends of the outer member first and second arms 220, 240 and the laterally-oriented surfaces 232. Similarly, a laterally-extending inferior exterior flat surface 216 of the outer member 210 is disposed between the distal ends of the outer member first and second arms 220, 240 and the laterally-oriented surfaces 232. The laterally-extending superior exterior flat surface 215 and the laterally-extending inferior exterior flat surface 216 are configured to serve as stops to prevent overarticulation of the implant 100 by abutting the superior and inferior first flat segments 123, 128 at one end of the articulation range and interacting with the superior and inferior second flat segments 124, 129 at the other end of the articulation range, as is described in detail below. The laterally-extending superior and inferior exterior flat surfaces 215, 216 also abut against the superior and inferior first flat segments 123, 128 of the implant 100, or against the superior and inferior second flat segments 124, 129 of the implant 100, during a portion of the implant insertion procedure.

The inner member 250 includes at its distal end a grasping portion 255 an inner member first arm 260 and an inner member second arm 280 separated by a split 270 that extends through the middle of the inner member 250 along the longitudinal axis from the grasping portion 255 toward the proximal end. The interior surface of the grasping portion 255 includes a plurality of engagement surfaces 257 that are configured to complementarily match the polygonal cross sectional geometry of the post 180 of the implant 100 and, thus, engage several of the plurality of facets 182a-182i. In a first preferred embodiment, there are seven engagement surfaces 257a-257g that are configured to engage seven of the nine facets 182a-182i of the post 180. Configured to interact with the interior surfaces of the outer member first and second arms 220, 240, the exterior surface of the inner member first arm 260 includes an inner member first arm exterior linear taper 262 disposed distal to an inner member first arm exterior straight portion 264, while the exterior surface of the inner member second arm 280 includes an inner member second arm exterior linear taper 282 disposed distal to an inner member second arm exterior straight portion 284. Disposed between the inner member first arm exterior linear taper 262 and the distal tip of the inner member first arm 260 is an inner member first arm second exterior linear taper 266.

Similarly, disposed between the inner member second arm exterior linear taper 282 and the distal tip of the inner member second arm 280 is an inner member second arm second exterior linear taper 286. Further, an inner member first arm laterally-oriented flat surface 265 and an inner member second arm laterally-oriented flat surface 285 are formed proximal to and adjacent the inner member first arm exterior straight portion 264 and the inner member second arm exterior straight portion 284, respectively, such that a pair of corners are formed therebetween, and such that the inner member first and second arm laterally-oriented flat surfaces 265, 285 face and abut with the laterally-oriented surfaces 232.

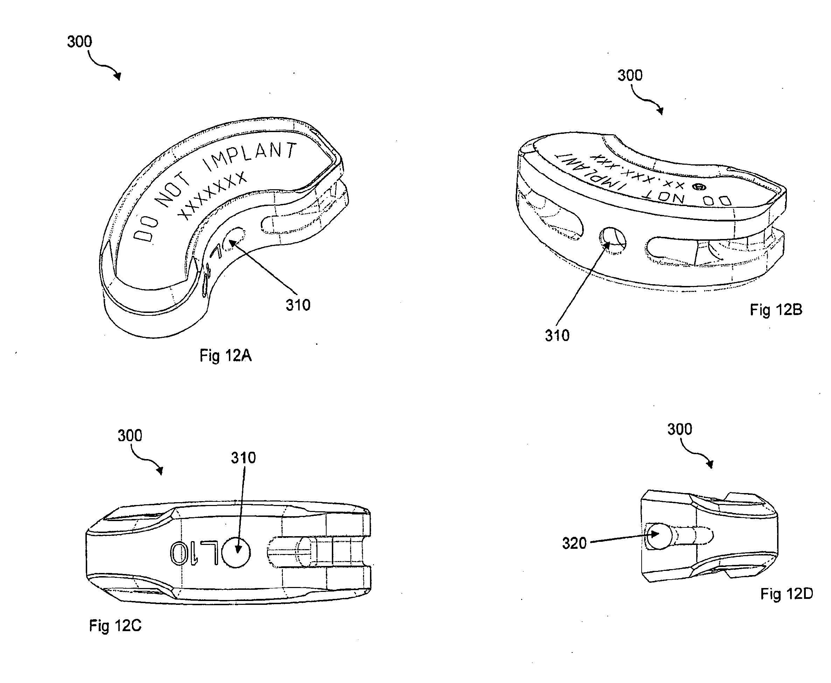

Referring to Fig. 12, a trial implant 300 is provided that includes geometry and surface features identical or similar to the implant 100 and further includes a lateral hole 310 and a longitudinal hole 320 and, therefore, a complete description of the trial implant is omitted for convenience only and is not limiting. The trial implant 300 is formed from a material that is visible under radiographic imaging, such as titanium, stainless steel, or the like. The lateral and longitudinal holes 310, 320, when viewed in conjunction with lateral and frontal X-rays, assist in the optimum positioning of the trial implant 300. The lateral holes 310 allow the surgeon to center the trial implant 300 with respect to the spinous processes of the vertebral bodies under fluoroscopy. The longitudinal hole 320 indicates whether the trial implant 300 has turned, in which case the surgeon will know that more disc material should preferably be removed. The lateral and longitudinal holes 310, 320 are shown as being generally circular or cylindrical in the preferred embodiment, but are not so limited. The lateral and longitudinal holes 310, 320 may have nearly any size and/or shape, such as rectangular, square, arrow-shaped, and/or triangular that permits visualization of the location of the trial implant 300 under imaging. In addition, the trial implant 300 is not limited to including the lateral and longitudinal holes 310, 320 or any holes, as location of the trial implant 300 may be visualized via markers or other features that are optically or machine viewable.

In operation, and in continuing reference to Figs. 1-12, a spinal disc in need of repair or replacement is identified and an at least partial discectomy is performed, preferably via a unilateral transforaminal approach. The trial implant 300 is inserted and removed using the instrument 200 to gauge the appropriate size implant 100 for insertion into the disc space. The insertion and manipulation of the trial implant 300 using the instrument 200 is identical to the method of inserting and manipulating the implant 100 using the instrument 200, as described below. The lateral and longitudinal holes 310, 320 are viewed using lateral and/or frontal X-rays to confirm the appropriate position of the trial implant 300 within the disc space and an implant size is then chosen.

Thus, the trial implant 300 is used for more than simply measuring the height between the vertebral bodies. Since the trial implant 300 articulates and is inserted to the same desired position as the final implant 100, the trial implant 300 may be used to determine whether the desired position of the implant 100 is reachable, whether enough disc material has been removed, and the like.

The bone graft 190 is then inserted into the axial bore 140 and secured therein via the surface features 145 (if not already preassembled thereto) and the implant 100 is then coupled to the instrument 200 by distracting the outer member 210 with respect to the inner member 250 via the manipulation of the actuation mechanism (not shown) such that the instrument 200 assumes an open configuration, as seen in Figs 7, 8, and 10C. The grasping portion 255 is then centered around the post 180 and the inner member 250 is partially retracted with respect to the outer member 210 via the manipulation of the actuation mechanism, thereby forcing the pair of corners formed between the inner member first and second arm exterior straight portions 264, 284 and the inner member first and second arm laterally-oriented flat surfaces 265, 285 to slidingly bear against the outer member first and second arm interior linear tapers 222, 242 until the inner member first and second arm exterior straight portions 264, 284 come to bear against the outer member first and second arm interior straight portions 224, 244, while providing the gap 230 between the inner member first and second arm laterally-oriented flat surfaces 265, 285 and the laterally-oriented surfaces 232. Consequently, the grasping portion 255 is collapsed around the post 180 such that the engagement surfaces 257a-g come into contact against the plurality of facets 182a-i of the post 180 and such that the post 180 is provisionally captured by the grasping portion 255, as shown in Fig. 9C, with the inner member first arm second exterior linear taper 286 bearing against the second linear surface 134.

In this provisionally locked configuration, the implant 100 is secured to the instrument but the post 180 is capable of rotation with respect to the grasping portion 255 but is prevented from exiting from the grasping portion 255. Final locking of the grasping portion 255 about the post 180, as shown in Figs. 9A, 9B, 10A, and 10B, is achieved by fully retracting the inner member 250 with respect to the outer member 210 via the continued manipulation of the actuation mechanism, thereby forcing the outer member first arm interior linear taper 222 and the outer member second arm interior linear taper 242 to come to bear against the inner member first arm exterior linear taper 262 and the inner member second arm exterior linear taper 282, respectively, thereby closing the gap 230, and finally locking the implant 100 to the instrument 200 while preventing any portions of the inner member first and second arms 260, 280 from separating under force from one another across the split 270 due to the contact between the outer member first and second arm interior linear tapers 222, 242 and the inner member first and second arm exterior linear tapers 262, 282. In this finally locked configuration, the superior and inferior exterior flat surfaces 215, 216 contact the superior and inferior first flat segments 123, 128, respectively, the gap 230 is closed, the inner member first arm second exterior linear taper 286 still bears against the second linear surface 134, and the post 180 is incapable of rotating with respect to the grasping portion 255.

In the finally locked configuration, the handle portion of the instrument 200 is grasped and the insertion end 110 of the implant is inserted into the transforaminal window created during the discectomy procedure until the bullet nose 112 enters the disc space and begins to distract the adjacent vertebral bodies and the distal end of the superior and inferior ridges 160, 165 make contact with the inferior surface of the superior vertebral body and the superior surface of the inferior vertebral body, respectively. Gentle hammer blows or other impaction forces are administered to the proximal end 201 of the instrument 200 to urge the implant 100 at least partially into the disc space. Toggling is prevented between the implant 100 and the instrument 200 during the delivery of impaction forces due to the abutment of (1) the superior and inferior first flat segments 123, 128 with the superior and inferior exterior flat surfaces 215, 216 and/or (2) the second linear surface 134 with the first arm second linear taper 286 and/or (3) the plurality of facets 182a-i of the post 180 with the engagement surface 257a-f when the instrument 200 is in its finally locked configuration with respect to the implant 100. Any of these abutments alone or in combination preferably prevent toggling between the implant 100 and the instrument 200 in the finally locked configuration.

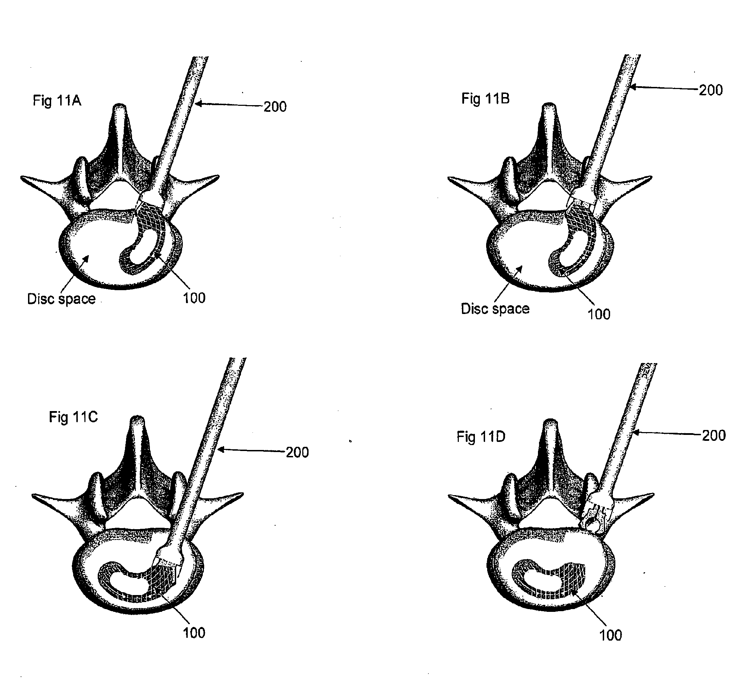

Once the impaction forces drive the implant 100 along a linear path to a desired position within the disc space, as seen in Fig. 11A, with the instrument 200 finally locked to the implant 100, the inner member 250 is advanced with respect to the outer member 210 such that the instrument reassumes its provisionally locked configuration with respect to the implant 100, in which the implant 100 is coupled to the instrument but the post 180 is capable of rotation with respect to the grasping portion 255. At this point, additional gentle hammer blows or other impaction forces are administered to the proximal end of the instrument 200 and the superior and inferior ridges 160, 165 contact the endplates of the vertebral bodies to promote turning of the implant 100 and guide the path of insertion of the implant 100 as the insertion end 110 progresses into the disc space. As the superior and inferior ridges 160, 165 guide the implant 100 into the desired position within the disc space, the post 180 and, hence, the implant 100, rotates with respect to the grasping portion 255 within a range restricted by the stops provided by the interaction between the inner member second arm second exterior linear taper 286 bearing against the second linear surface 134 (the starting configuration of the insertion method) and the inner member first arm second exterior linear taper 266 bearing against the first linear surface 132 (at maximum angulation).

Throughout the entirety of the insertion process, the angle of the shaft of the instrument 200 with respect to the disc space is maintained constant, as all of the action performed to articulate the implant 100 is undertaken by the implant 100 itself as the gentle impaction forces drive the implant 100 into its desired final position guided by the superior and inferior ridges 160, 165, with no active turning of the implant necessary. Upon contact between the inner member first arm second exterior linear taper 266 and the first linear surface 132, the implant 100 is at or near its desired final positioning interior to the disc space. At this point, the implant 100 can be repositioned as necessary by again finally locking the implant 100 to the instrument 200, by retracting the inner member 250 distally with respect to the outer member 210, and manipulating the handle of the instrument 200 until the optimum final positioning of the implant 100 is achieved with respect to the disc space while viewing the position of the markers 170 under fluoroscopic imaging. The arrangement of the markers 170 enables a single radiographic image, e.g., a lateral image, to be used to determine the precise position of the implant 100 with respect to the disc space. The implant 100 is then released from the instrument 200 by manipulating the actuation mechanism until the instrument 200 assumes its open configuration, as described previously, and the grasping portion 255 no longer contacts the post 180. The compression forces between the vertebral endplates and the superior and inferior surfaces 120, 125 maintain the implant 100 in place as the instrument 200 is removed from the disc space and the patient's body.

The insertion and removal of the trial implant 300 may cause the formation of grooves in the adjacent endplates of the superior and inferior vertebral bodies due to the inclusion on the superior and inferior surfaces of the trial implant 300 of superior and inferior ridges that are identical to the superior and inferior ridges 160, 165 of the implant 100. The formation of such grooves in the adjacent endplates of the superior and inferior vertebral bodies, while not required for insertion of the implant 100, may assist in easing the insertion of the implant 100 using the instrument 200 via the guided mating of the superior and inferior ridges 160, 165 with the grooves formed previously by the trial implant 300.

While embodiments of the present invention are described herein with respect to an interbody spacer configured for insertion via a transforaminal path, a variety of implants may be utilized, such as total disc replacements and nucleus replacement devices, by simply configuring such implants to include an appropriately faceted post for an instrument engagement feature and, optionally, the stops and toggle-free bearing surfaces described herein. As such, the implant 100 is not limited to a banana or kidney bean shape, but may assume any geometry that can be accommodated within the disc space. Further, a range of angular approaches to the disc space may be utilized where an elongated implant is desired to be manipulated or pivoted once it has been delivered along a straight path into the disc space, such as posterior-lateral approaches, translateral, and direct lateral procedures.

In an alternate embodiment, the non-toggling interface between the implant 100 and the instrument 200 during the delivery of impaction forces that is provided by the interaction and abutment of the superior and inferior first flat segments 123,128 with the laterally-extending superior and inferior exterior flat surfaces 215, 216, as well as the interaction and abutment of the superior and inferior second flat segments 124, 129 with the laterally-extending superior and inferior exterior flat surfaces 215, 216, can also be provided with nonlinear abutment surfaces. As long as the surfaces mate or are able to abut one another when the instrument assumes its finally locked configuration, a non-toggling interface can be provided.

Similarly, the articulation stops that prevent overarticulation of the implant 100 with respect to the instrument 200 that are embodied by the first and second linear surfaces 132, 134, and the range of articulation provided by the obtuse angle disposed therebetween, can be provided by a variety of angles which can be tailored specifically to a desired articulation range for a given application, and therefore does not necessarily need to be obtuse. Further, the first and second linear surfaces 132, 134, as well as the inner member first and second arm second exterior linear tapers 266, 286 that are abutted thereagainst, need not be linear surfaces. Rather, any mating abutment surfaces will suffice between 132 and 266 and between 134 and 286 for the purposes of limiting the articulation range. Further, an embodiment may be envisioned in which the obtuse angle is removed between the first and second linear surfaces 132, 134 such that a single abutment surface is provided that can limit the range of articulation by being abuttable by both the first and second arm second exterior linear tapers 266, 286 and, further, does not need to be linear as long as it provides a mating abutment surface to the geometry chosen for the first and second arm second exterior linear tapers 266, 286.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the scope of the present invention as defined by the present description.

## Claims

1. An intervertebral implant (100) comprising:
(a) an insertion end (110) and an opposing engagement end (115);
(b) first and second opposed main surfaces (120, 125) configured to contact respective adjacent vertebral endplates, each of the first and second main surfaces (120, 125) having an anterior edge, a posterior edge, and extending between the insertion and engagement ends (110, 115);
(c) an anterior wall (130) formed between the first and second main surfaces (120, 125) and along the anterior edges thereof;
(d) a posterior wall (130) formed between the first and second main surfaces (120, 125) and along the posterior edges thereof, the anterior wall (130) and the posterior wall (130) converging at the insertion and engagement ends (110, 115); and
(e) a slot (135) formed at the engagement end (115) and extending continuously between and at least partially along the anterior and posterior walls (130), a post (180) positioned within the slot (135), spaced from at least one of the anterior and posterior walls (130) and extending at least partially between the first and second main surfaces (120, 125),
**characterized in that** the post (180) includes a plurality of exposed facets (182a - 182i), and **in that** the post (180) is configured for pivotable engagement with an insertion instrument (200).

2. The intervertebral implant of claim 1, wherein each anterior edge is generally concave and each posterior edge is generally convex.

3. The intervertebral implant of claim 2, wherein each of the first and second main surfaces (120, 125) includes a plurality of curved parallel ridges (160, 165) protruding from the respective surface and extending from the insertion end (110) to the engagement end (115), each of the plurality of parallel ridges (160, 165) including a plurality of teeth (162, 167).

4. The intervertebral implant of any one of claims 1 to 3, wherein the anterior and posterior walls define a first height of the implant (100) proximate the engagement end (115) and a second height at the insertion end (110), the second height being less than the first height.

5. The intervertebral implant of any one of claims 1 to 4, wherein each anterior edge has a generally linear portion (124, 129) proximate the engagement end (115) and a generally concave portion, and each posterior edge has a generally linear portion (123, 128) proximate the engagement end (115) and a generally convex portion, the generally linear portion (124, 129) of the anterior edge converging with the generally linear portion (123, 128) of the posterior edge at the engagement end (115) for each of the first and second main surfaces (120, 125).

6. The intervertebral implant of any one of claims 1 to 5, wherein
each anterior edge is generally concave and each posterior edge is generally convex,
the facets (182a-182i) are disposed around an entire periphery of the post (180), and
at least one abutment surface (132, 134) is disposed within the slot (135) distally from the post (180), the at least one abutment surface (132, 134) limiting rotation of the implant (100) about the post (180) when the post (180) is engaged with the pivotable insertion instrument (200).

7. The intervertebral implant of any one of claims 1 to 6, wherein
each anterior edge is generally concave and each posterior edge is generally convex, an axial bore (140) being formed between the anterior and posterior edges and extending between the first and second main surfaces (120, 125);
the implant further comprising:
(f) a plurality of markers (170), at least one of the markers (170 extending between the first and second main surfaces (120, 125) within one of the anterior and posterior walls (130), at least one other of the markers (170) being disposed generally transverse to the at least one of the markers (170) and extending from the insertion end (110) toward the axial bore (140).

8. The implant of any one of claims 1 to 7, wherein the post (180) is polygonal in cross-section and includes nine exposed facets (182a - 182i) arranged around an entire periphery thereof and extending between the first and second opposed main surfaces (120, 125).

9. The implant of claim 8, wherein the post (180) includes nine exposed facets (182a - 182i), seven of the facets (182a - 182f, 182i) being flat surfaces, while the remaining two facets (182g - 182h) being curved surfaces.

10. A system for spine surgery at a disc space disposed between first and second endplates of adjacent vertebral bodies of a patient, the system comprising:
(a) an intervertebral implant (100) of any one of claims 1 to 9;
(b) a trial implant (300) including:
(i) an insertion end and an opposing engagement end,
(ii) first and second opposed main surfaces configured to contact respective adjacent vertebral endplates, each of the first and second main surfaces having an anterior edge, a posterior edge, and extending between the insertion and engagement ends,
(iii) an anterior wall formed between the first and second main surfaces and along the anterior edges thereof,
(iv) a posterior wall formed between the first and second main surfaces and along the posterior edges thereof, the anterior wall and the posterior wall converging at the insertion and engagement ends, and
(v) a slot formed at the engagement end and extending continuously between and at least partially along the anterior and posterior walls, a post positioned within the slot, spaced from at least one of the anterior and posterior walls and extending at least partially between the first and second main surfaces, the post including a plurality of exposed facets; and
(c) an insertion instrument (200) including:
(i) a proximal end, a distal end, and a longitudinal axis therebetween,
(ii) an inner member (250) and an outer member (210), the inner member (250) being translatable with respect to the outer member (210) along the longitudinal axis and having a grasping portion (255) at the distal end, the grasping portion including a plurality of facet surfaces (257a - 257g) engagable with the plurality of the post facets (182a- 182i) of the intervertebral implant (100) and the trial implant (200),
the instrument (200) having a first configuration in which the grasping portion (255) assumes an open configuration for allowing coupling of the instrument (200) to the post (180) of one of the intervertebral implant (100) and the trial implant (300), a second configuration in which the instrument (200) is securely coupled to the post (180) of one of the intervertebral implant (100) and the trial implant (300) while allowing the post (180) to rotate within the grasping portion (255) under a given force, and a third configuration wherein the instrument (200) is securely coupled to the post (180) of one of the intervertebral implant (100) and the trial implant (300) while preventing rotation of the post (180) with respect to the grasping portion (255).

## Patentansprüche

1. Zwischenwirbelimplantat (100), umfassend:
(a) ein Einführende (110) und ein gegenüberliegendes Eingriffsende (115),
(b) eine erste und zweite gegenüberliegende Hauptoberfläche (120, 125), die eingerichtet sind, entsprechende benachbarte Wirbelkörperendplatten zu kontaktieren, wobei jede der ersten und zweiten Hauptoberfläche (120, 125) einen anterioren Rand, einen posterioren Rand aufweist und sich zwischen dem Einführ- und Eingriffsende (110, 115) erstreckt,
(c) eine anteriore Wand (130), die zwischen der ersten und zweiten Hauptoberfläche (120, 125) und entlang deren anterioren Rändern gebildet ist,
(d) eine posteriore Wand (130), die zwischen der ersten und zweiten Hauptoberfläche (120, 125) und entlang deren posterioren Rändern gebildet ist, wobei die anteriore Wand (130) und die posteriore Wand (130) an dem Einführ- und Eingriffsende (110,115) konvergieren, und
(e) einen Schlitz (135), der an dem Eingriffsende (115) gebildet ist und sich kontinuierlich zwischen und zumindest teilweise entlang der anterioren und posterioren Wand (130) erstreckt, wobei eine Säule (180) in dem Schlitz (135) positioniert ist, die von zumindest einer der anterioren und posterioren Wand (130) beabstandet ist und sich zumindest teilweise zwischen der ersten und zweiten Hauptoberfläche (120, 125) erstreckt,
**dadurch gekennzeichnet, dass** die Säule (180) eine Mehrzahl von freiliegenden Facetten (182a - 182i) enthält, und dadurch, dass die Säule (180) für einen schwenkbaren Eingriff mit einem Einführinstrument (200) eingerichtet ist.

2. Zwischenwirbelimplantat nach Anspruch 1, wobei jeder anteriore Rand im Allgemeinen konkav ist und jeder posteriore Rand im Allgemeinen konvex ist.

3. Zwischenwirbelimplantat nach Anspruch 2, wobei jede der ersten und zweiten Hauptoberfläche (120, 125) eine Mehrzahl von gekrümmten parallelen Rippen (160, 165) enthält, die von der entsprechenden Oberfläche hervorragen und sich von dem Einführende (110) zu dem Eingriffsende (115) erstrecken, wobei jede der Mehrzahl von parallelen Rippen (160, 165) eine Mehrzahl von Zähnen (162, 167) enthält.

4. Zwischenwirbelimplantat nach einem der Ansprüche 1 bis 3, wobei die anteriore und posteriore Wand eine erste Höhe des Implantats (100) nahe dem Eingriffsende (115) und eine zweite Höhe an dem Einführende (110) definieren, wobei die zweite Höhe geringer ist als die erste Höhe.

5. Zwischenwirbelimplantat nach einem der Ansprüche 1 bis 4, wobei jeder anteriore Rand einen im Allgemeinen linearen Teil (124, 129) nahe dem Eingriffsende (115) und einen im Allgemeinen konkaven Teil aufweist und jeder posteriore Rand einen im Allgemeinen linearen Teil (123, 128) nahe dem Eingriffsende (115) und einen im Allgemeinen konvexen Teil aufweist, wobei der im Allgemeinen lineare Teil (124, 129) des anterioren, Rands mit dem im Allgemeinen linearen Teil (123, 128) des posterioren Rands an dem Eingriffsende (115) für jede der ersten und zweiten Hauptoberfläche (120, 125) konvergiert.

6. Zwischenwirbelimplantat nach einem der Ansprüche 1 bis 5, wobei
jeder anteriore Rand im Allgemeinen konkav und jeder posteriore Rand im Allgemeinen konvex ist,
sich die Facetten (182a - 182i) um einen gesamten Umfang der Säule (180) herum befinden und
sich zumindest eine Anlageoberfläche (132,134) in dem Schlitz (135) distal von der Säule (180) befindet, wobei die zumindest eine Anlageoberfläche (132,134) eine Drehung des Implantats (100) um die Säule (180) herum begrenzt, wenn die Säule (180) mit dem schwenkbaren Einführinstrument (200) in Eingriff ist.

7. Zwischenwirbelimplantat nach einem der Ansprüche 1 bis 6, wobei
jeder anteriore Rand im Allgemeinen konkav und jeder posteriore Rand im Allgemeinen konvex ist,
eine axiale Bohrung (140) zwischen dem anterioren und posterioren Rand gebildet ist und sich zwischen der ersten und zweiten Hauptoberfläche (120, 125) erstreckt,
wobei das Implantat des Weiteren umfasst:
(f) eine Mehrzahl von Markern (170), wobei sich zumindest einer der Marker (170) zwischen der ersten und zweiten Hauptoberfläche (120, 125) in einer der anterioren und posterioren Wand (130) erstreckt, wobei sich zumindest ein anderer der Marker (170) im Allgemeinen quer zu dem einen der Marker (170) befindet und sich von dem Einführende (110) zu der axialen Bohrung (140) erstreckt.

8. Implantat nach einem der Ansprüche 1 bis 7, wobei die Säule (180) im Querschnitt polygonal ist und neun freiliegende Facetten (182a - 182i) enthält, die um deren gesamten Umfang herum angeordnet sind und sich zwischen der ersten und zweiten gegenüberliegenden Hauptoberfläche (120, 125) erstrecken.

9. Implantat nach Anspruch 8, wobei die Säule (180) neun freiliegende Facetten (182a - 182i) enthält, wobei sieben der Facetten (182a - 182f, 182i) flache Oberflächen sind, während die restlichen zwei Facetten (182g - 182h) gekrümmte Oberflächen sind.

10. System für Wirbelsäulenchirurgie in einem Scheibenraum, der sich zwischen einer ersten und zweiten Endplatte benachbarter Wirbelkörper eines Patienten befindet, wobei das System umfasst:
(a) ein Zwischenwirbelimplantat (100) nach einem der Ansprüche 1 bis 9,
(b) ein Probeimplantat (300), enthaltend:
(i) ein Einführende und ein gegenüberliegendes Engriffsende,
(ii) eine erste und zweite gegenüberliegende Hauptoberfläche, die eingerichtet sind, entsprechende benachbarte Wirbelkörperendplatten zu kontaktieren, wobei jede der ersten und zweiten Hauptoberfläche einen anterioren Rand, einen posterioren Rand aufweist und sich zwischen dem Einführ- und Eingriffsende erstreckt,
(iii) eine anteriore Wand, die zwischen der ersten und zweiten Hauptoberfläche und entlang deren anterioren Rändern gebildet ist,
(iv) eine posteriore Wand, die zwischen der ersten und zweiten Hauptoberfläche und entlang deren posterioren Rändern gebildet ist, wobei die anteriore Wand und die posteriore Wand an dem Einführ- und Eingriffsende konvergieren, und
(v) einen Schlitz, der an dem Eingriffsende gebildet ist und sich kontinuierlich zwischen und zumindest teilweise entlang der anterioren und posterioren Wand erstreckt, wobei eine Säule in dem Schlitz positioniert ist, die von zumindest einer der anterioren und posterioren Wand beabstandet ist und sich zumindest teilweise zwischen der ersten und zweiten Hauptoberfläche erstreckt, wobei die Säule eine Mehrzahl von freiliegenden Facetten enthält, und
(c) ein Einführinstrument (200), enthaltend:
(i) ein proximales Ende, ein distales Ende und eine Längsachse dazwischen,
(ii) ein inneres Bauteil (250) und ein äußeres Bauteil (210), wobei das innere Bauteil (250) bezüglich des äußeren Bauteils (210) entlang der Längsachse verschiebbar ist und einen Greifteil (255) an dem distalen Ende aufweist, wobei der Greifteil eine Mehrzahl von Facettenoberflächen (257a - 257g) enthält, die mit der Mehrzahl der Säulenfacetten (182a - 182i) des Zwischenwirbelimplantats (100) und des Probeimplantats (200) in Eingriff bringbar sind,
wobei das Instrument (200) eine erste Konfiguration aufweist, in welcher der Greifteil (255) eine offene Konfiguration annimmt, um ein Koppeln des Instruments (200) mit der Säule (180) eines des Zwischenwirbelimplantats (100) und des Probeimplantats (300) zu erlauben, eine zweite Konfiguration, in welcher das Instrument (200) sicher mit der Säule (180) eines des Zwischenwirbelimplantats (100) und des Probeimplantats (300) gekoppelt ist, während es der Säule (180) erlaubt ist, in dem Greifteil (255) unter einer vorgegebenen Kraft zu drehen, und eine dritte Konfiguration, wobei das Instrument (200) sicher mit der Säule (180) eines des Zwischenwirbelimplantats (100) und des Probeimplantats (300) gekoppelt ist, während eine Drehung der Säule (180) bezüglich des Greifteils (255) verhindert wird.

## Revendications

1. Implant intervertébral (100) comprenant :
(a) une extrémité d'introduction (110) et une extrémité de prise opposée (115) ;
(b) des première et seconde surfaces principales opposées (120, 125) configurées pour être en contact avec des plateaux d'extrémité vertébraux adjacents, chacune des première et seconde surfaces principales (120, 125) présentant un bord antérieur, un bord postérieur et s'étendant entre les extrémités d'introduction et de prise (110, 115) ;
(c) une paroi antérieure (130) formée entre les première et seconde surfaces principales (120, 125) et le long des bords antérieurs de celles-ci ;
(d) une paroi postérieure (130) formée entre les première et seconde surfaces principales (120, 125) et le long des bords postérieurs de celles-ci, la paroi antérieure (130) et la paroi postérieure (130) convergeant au niveau des extrémités d'introduction et de prise (110, 115), et
(e) une fente (135) formée au niveau de l'extrémité de prise (115) et s'étendant de manière continue entre des parois antérieure et postérieure (130), et au moins en partie le long de celles-ci, un tenon (180) positionné à l'intérieur de la fente (135), à distance d'au moins l'une des parois antérieure et postérieure (130) et s'étendant au moins en partie entre les première et seconde surfaces principales (120, 125),
**caractérisé en ce que** le tenon (180) inclut une pluralité de facettes exposées (182a-182i) et **en ce que** le tenon (180) est configuré pour une prise pivotable avec un instrument d'introduction (200).

2. Implant intervertébral selon la revendication 1, dans lequel chaque bord antérieur est généralement concave, et chaque bord postérieur est généralement convexe.

3. Implant intervertébral selon la revendication 2, dans lequel chacune des première et seconde surfaces principales (120, 125) inclut une pluralité d'arêtes parallèles incurvées (160, 165) faisant saillie à partir de la surface respective et s'étendant de l'extrémité d'introduction (110) à l'extrémité de prise (115), chacune de la pluralité d'arêtes parallèles (160, 165) incluant une pluralité de dents (162, 167).

4. Implant intervertébral selon l'une quelconque des revendications 1 à 3, dans lequel les parois antérieure et postérieure définissent une première hauteur de l'implant (100) à proximité de l'extrémité de prise (115) et une seconde hauteur au niveau de l'extrémité d'introduction (110), la seconde extrémité étant inférieure à la première hauteur.

5. Implant intervertébral selon l'une quelconque des revendications 1 à 4, dans lequel chaque bord antérieur présente une partie généralement linéaire (24, 129) à proximité de l'extrémité de prise (115) et une partie généralement concave, et chaque bord postérieur présente une partie généralement linéaire (123, 128) à proximité de l'extrémité de prise (115) et une partie généralement convexe, la partie généralement linéaire (124, 129) du bord antérieur convergeant avec la partie généralement linéaire (123, 128) du bord postérieur au niveau de l'extrémité de prise (115) pour chacune des première et seconde surfaces principales (120, 125).

6. Implant intervertébral selon l'une quelconque des revendications 1 à 5, dans lequel
chaque bord antérieur est généralement concave et chaque bord postérieur est généralement convexe ;
les facettes (182a-182i) sont disposées autour de la périphérie toute entière du tenon (180), et
au moins une surface d'about (132, 134) est disposée à l'intérieur de la fente (135) de manière distale par rapport au tenon (180) ; la au moins une surface d'about (132, 134) limitant la rotation de l'implant (100) autour du tenon (180) lorsque le tenon (180) est en prise avec l'instrument d'introduction pivotable (200).

7. Implant intervertébral selon l'une quelconque des revendications 1 à 6, dans lequel
chaque bord antérieur est généralement concave et chaque bord postérieur est généralement convexe, un alésage axial (140) étant formé entre les bords antérieur et postérieur et s'étendant entre les première et seconde surfaces principales (120 ; 125) ;
l'implant comprenant en outre :
(f) une pluralité de marqueurs (170), au moins un des marqueurs (170) s'étendant entre les première et seconde surfaces principales (120, 125) à l'intérieur d'une des parois antérieure et postérieure (130), au moins un autre des marqueurs (170) étant disposé généralement transversalement au au moins un des marqueurs (170) et s'étendant de l'extrémité d'introduction (110) vers l'alésage axial (140).

8. Implant selon l'une quelconque des revendications 1 à 7, dans lequel le tenon (180) est de section polygonale et inclut neuf facettes exposées (182a-182i) agencées autour de la périphérie toute entière de celui-ci et s'étendant entre les première et seconde surfaces principales opposées (120, 125).

9. Implant selon la revendication 8, dans lequel le tenon (180) inclut neuf facettes exposées (182a-182i), sept des facettes (182a-182f, 182i) étant des surfaces plates, tandis que les deux facettes restantes (182g-182h) sont des surfaces incurvées.

10. Système pour chirurgie de la colonne vertébrale au niveau d'un espace de disque disposé entre des premier et second plateaux d'extrémité de corps vertébraux adjacents d'un patient, le système comprenant :
(a) un implant intervertébral (100) selon l'une quelconque des revendications 1 à 9 ;
(b) un implant d'essai (300) incluant :
(i) une extrémité d'introduction et une extrémité de prise opposée ;
(ii) des première et seconde surfaces principales opposées configurées pour être en contact avec des plateaux d'extrémité vertébraux adjacents, chacune des première et seconde surfaces principales présentant un bord antérieur, un bord postérieur et s'étendant entre les extrémités d'introduction et de prise ;
(iii) une paroi antérieure formée entre les première et seconde surfaces principales et le long des bords antérieurs de celles-ci ;
(iv) une paroi postérieure formée entre les première et seconde surfaces principales et le long des bords postérieurs de celles-ci, la paroi antérieure et la paroi postérieure convergeant au niveau des extrémités d'introduction et de prise, et
(v) une fente formée au niveau de l'extrémité de prise et s'étendant de manière continue entre les parois antérieure et postérieure, et au moins en partie le long de celles-ci, un tenon positionné à l'intérieur de la fente, à distance d'au moins l'une des parois antérieure et postérieure et s'étendant au moins en partie entre les première et seconde surfaces principales, le tenon (180) incluant une pluralité de facettes exposées, et
(c) un instrument d'introduction (200) incluant :
(i) une extrémité proximale, une extrémité distale et un axe longitudinal entre celles-ci ;
(ii) un élément intérieur (250) et un élément extérieur (210), l'élément intérieur (250) pouvant subir une translation par rapport à l'élément extérieur (210) le long de l'axe longitudinal et présentant une partie de préhension (255) au niveau de l'extrémité distale, la partie de préhension incluant une pluralité de surfaces à facettes (257a-257g) pouvant venir en prise avec la pluralité de facettes de tenon (182a-182i) de l'implant intervertébral (100) et de l'implant d'essai (200),
l'instrument (200) présentant une première configuration dans laquelle la partie de préhension (255) adopte une configuration ouverte pour permettre un couplage de l'instrument (200) avec le tenon (180) d'un parmi l'implant intervertébral (100) et l'implant d'essai (300), une deuxième configuration dans laquelle l'instrument (200) est couplé solidement avec le tenon (180) de l'un parmi l'implant intervertébral (100) et l'implant d'essai (300) tout en permettant au tenon (180) de tourner à l'intérieur de la partie de préhension (255) sous une force donnée, et une troisième configuration dans laquelle l'instrument (200) est couplé solidement avec le tenon (180) d'un parmi l'implant intervertébral (100) et l'implant d'essai (300) tout en empêchant la rotation du tenon (180) par rapport à la partie de préhension (255).
